# EUROPÄISCHE PATENTANMELDUNG

(11) **EP 1 283 049 A1**
(43) Veröffentlichungstag der Anmeldung: **12.02.2003**
(21) Anmeldenummer: 02017592.3
(22) Anmeldetag: 07.08.2002
(51) Int. Cl.: A61K 38/00, A61K 38/16, A61K 38/44, A61K 38/48, A61P 35/00, A61K 35/74

(54) **Verarmung des Lysin-Pools zur Suppression maligner Proliferationsprozesse**

(30) Priorität: 08.08.2001 DE 10139039
(71) Anmelder: Douvlis, Zinon, Dr.med., 27628 Sandstedt (DE)
(72) Erfinder: Douvlis, Zinon, Dr.med., 27628 Sandstedt (DE)
(74) Vertreter: Läufer, Martina, Dr.

(57) **Zusammenfassung**

Die Reduktion der Verfügbarkeit der Aminosäure Lysin korreliert mit einer Suppression maligner Proliferationsprozesse. Maligne Tumorzellen sezernieren eine Retrograddifferenzierte-Muskel-Degradations-Protease (RMDP), deren Ziel die Degradation der Proteine der Skelettmuskulatur ist. Die RMDP kann als fehlerhaftes Protein vom Wirtsorganismus nicht effektiv abgebaut werden. Die Reduktion der Verfügbarkeit von Lysin als für die RMDP-Synthese wesentlichen Aminosäure führt zu einem Antitumoreffekt. Es werden verschiedene Mittel für die Reduktion der Verfügbarkeit des Lysin angegeben.

## Beschreibung

Die Erfindung betrifft die Verwendung von Mitteln zur Suppression maligner Proliferationsprozesse mit einem Antitumoreffekt, die daher in der Krebstherapie verwendet werden können, sowie zugehörige hierfür verwendbare Zubereitungen.

Der Pathogenitätsmechanismus von malignen Proliferationsprozessen basiert auf der Perpetuisierung einer - im Verhältnis zum eigenen Bedarf der malignen Tumorzellen - übermäßigen Freisetzung von Aminosäuren durch Abbauprozesse der Proteine der Skelettmuskulatur.

Unter diesem Aspekt ist die Zielsetzung eines Therapiekonzeptes, die Perpetuisierung der Freisetzung von Aminosäuren zu blockieren. Die Aufgabe der Erfindung besteht darin, hierfür Mittel zur Verfügung zu stellen.

Diese Aufgabe wird erfindungsgemäß durch die Merkmale des Anspruchs 1 gelöst. Weitere Ausführungsformen sind in den Unteransprüchen gekennzeichnet.

Die Hemmung des Abbaus der oben genannten Proteine ist von einem Antitumoreffekt begleitet. Der enzymatische Abbau von Muskelproteinen erfolgt durch ein von den malignen Tumorzellen initiiertes bzw. produziertes, als Muskel-Protein-Degradations-Enzym zu bezeichnendes Enzym.

Entsprechend des Differenzierungsniveaus der malignen Tumorzellen ist das vorgenannte Enzym als eine retrograddifferenzierte Muskel-Degradations-Protease zu bezeichnen, die der Kontrolle der Enzymregulation des Wirtsorganismus nicht unterworfen ist.

Ein Außerkraftsetzen des vom Muskel-Protein-Degradations-Enzym abhängigen Pathogenitätsmechanismus kann
1. durch Suppression der Genexpression, die für die Synthese des Enzyms, das für die enzymatische Freisetzung von Aminosäuren aus der Skelettmuskulatur verantwortlich ist,
realisiert werden.

Die Zielsetzung der Erfindung kann 2. durch eine Kombination von Enzymaktivitäten mit prae- und post-Synthese-Orientierung, die mit einem Antitumor- bzw. Malignitäts-Suppressor-Effekt assoziieren, bzw. durch die Gabe medikamentös wirkender Kombinationen entsprechender Mittel realisiert werden.
2.1. Durch Hemmung der Synthese des Muskel-Protein-Degratations-Enzyms durch Verarmung des Pools bzw. durch Reduktion der Verfügbarkeit der Aminosäuren, die für die Synthese des oben genannten Enzyms von Bedeutung sind. Die Reduktion der Verfügbarkeit der Aminosäure Lysin, die von Bedeutung für die Synthese des oben genannten Enzyms ist, wird von einem Antitumoreffekt begleitet.

Die Reduktion der Verfügbarkeit von Lysin kann durch verschiedenen Enzyme erreicht werden:
Geeignet sind insbesondere Lysin-Ketoglutarsäure (Lysin-Ketoglutaric acid, LKR, Lysin-Enzym des Saccharopin Weges in Pflanzen);

Saccharopin-Dehydrogenase (SDH Enzym des Saccharopin-Weges in Pflanzen; sowohl LKR als auch SDH kommen in Mais vor);

Lysyl-Oxydase, die Lysin in Alysin umwandelt.

Die Reduktion der Verfügbarkeit von Lysin kann ferner durch Bindung von Lysin an ein Lysin-bindendes Protein aus Pichia pastoris erreicht werden.

Die Reduktion der Verfügbarkeit kann ferner durch Lysinantagonisten, z.B. L-Canalin erzielt werden.

Neben der vorgenannten Verwendung von Mitteln zur Verarmung des Lysin-Pools in Zellen können Mittel zur Spaltung von Lysinbindungen in Proteinen oder Peptiden genutzt werden. Die Suppression kann daher bewirkt werden durch:
2.2 Inaktivierung des Muskel-Protein-Degradations-Enzyms. Diese ist durch enzymatische Spaltung von Bindungen derjenigen Aminosäuren zu erreichen, die für die Synthese und den Funktionszustand von Bedeutung sind. Für die Spaltung von Lysinbindungen, die von einem Antitumoreffekt begleitet werden, sei beispielhaft genannt, die Spaltung von Lysinbindungen durch die Arylaminopeptidase von Streptococcus sanguis.

## Patentansprüche

1. Verwendung von Mitteln zur Verarmung des Lysin-Pools in Zellen und/oder von Mitteln zur Spaltung von Lysin-Bindungen in Proteinen oder Peptiden zur Suppression maligner Proliferationsprozesse in der Krebstherapie.

2. Verwendung nach Anspruch 1, **dadurch gekennzeichnet, dass** die Mittel zur Verarmung des Lysin-Pools Enzyme sind, insbesondere Lysin-Enzyme, wie insbesondere Lysin-Ketoglutarsäure, Saccharopin-Dehydrogenase, Lysyl-Oxydase, Lysin-bindende Substanzen, insbesondere ein Lysin-bindendes Protein aus *Pichia pastoris*, oder Lysin-Antagonisten, insbesondere L-Canalin.

3. Verwendung nach Anspruch 1 oder 2, **dadurch gekennzeichnet, dass** die Mittel zur Spaltung von Lysin-Bindungen in Proteinen oder Peptiden, Enzyme, insbesondere Peptidasen sind.

4. Verwendung nach Anspruch 2, **dadurch gekennzeichnet, dass** die Peptidase eine Arylaminopeptidase, insbesondere von Streptococcus sanguis ist.

5. Verwendung von Streptococcus sanguis zur Suppression maligner Proliferationsprozesse in der Krebstherapie.

6. Zubereitung zur medikamentösen Verwendung in der Krebstherapie, **gekennzeichnet durch** einen Gehalt an Streptococcus sanguis.

7. Zubereitung zur medikamentösen Verwendung in der Krebstherapie, **gekennzeichnet durch** einen Gehalt an Lysin-spaltenden Peptidasen, insbesondere Arylaminopeptidasen, und/oder Lysin-Enzymen, insbesondere Lysin-Ketoglutarsäure, Saccharopin-Dehydrogenase, Lysyl-Oxydase, und/oder Lysin-bindenden Substanzen, insbesondere einem Lysin-bindenden Protein aus *Pichia pastoris,* und/oder Lysin-Antagonisten, insbesondere L-Canalin.
